# EUROPÄISCHE PATENTSCHRIFT

(11) **EP 0 788 337 B1**
(45) Veröffentlichungstag und Bekanntmachung des Hinweises auf die Patenterteilung: **15.03.2000**
(21) Anmeldenummer: 95935402.8
(22) Anmeldetag: 28.09.1995
(51) Int. Cl.: A61F 13/50

(54) **HYGIENEPRODUKT MIT FASERIGEM SAUGKÖRPER**
HYGIENE PRODUCT WITH A FIBROUS ABSORBER
ARTICLE D'HYGIENE A CORPS ABSORBANT FIBREUX

(30) Priorität: 25.10.1994 DE 4437992
(43) Veröffentlichungstag der Anmeldung: 13.08.1997
(73) Patentinhaber: Paul Hartmann Aktiengesellschaft, 89522 Heidenheim (DE)
(72) Erfinder: HERMANN, Klaus, D-89537 Giengen (DE); WURSTER, Thomas, D-89522 Heidenheim (DE); MALOWANIEC, Krzysztof, D., D-89522 Heidenheim (DE)
(74) Vertreter: Dreiss, Fuhlendorf, Steimle & Becker, Patentanwälte
(86) Internationale Anmeldenummer: EP9503840
(87) Internationale Veröffentlichungsnummer: WO9612463

(56) Entgegenhaltungen:
- EP-A- 0 291 316
- EP-A- 0 304 957
- EP-A- 0 494 112
- AU-B- 512 002
- US-A- 3 965 904

## Beschreibung

Die Erfindung betrifft ein Hygieneprodukt mit einem Fasern enthaltenden und Bereiche unterschiedlicher Dichte aufweisenden Saugkörper.

Bekannte Hygieneprodukte, wie beispielsweise Wegwerfwindeln, Monatsbinden, Inkontinenzprodukte oder sonstige flüssigkeitsaufsaugende Unterlagen, weisen in der Regel einen Saugkörper auf. Dieser Saugkorper besteht zumeist im wesentlichen aus Fasern, wie Zellulosefasern, die auf eine bestimmte Dichte verdichtet sind, um dem Saugkörper einen gewissen Zusammenhalt zu geben. Das Vermögen des Saugkörpers Flüsigkeit aufzusaugen und zu halten ist dabei stark abhängig von der Dichte des Saugkörpers. Ein Saugkörper höherer Dichte kann aufgrund der größeren Kapillarwirkung, die durch den geringeren Abstand der Fasern zueinander entsteht, die anfallende Flüssigkeit schneller aufsaugen als ein weniger verdichteter Saugkörper. Andererseits kann der höher verdichtete Saugkörper weniger Flüssigkeit speichern, da die Faserzwischenräume kleiner sind.

Diese Effekte wurden bei der Entwicklung von Saugkörpern ausgenutzt und so die verschiedensten Saugkörper für Hygieneprodukte entwickelt, wobei die Saugkörper zur Optimierung der Flüssigkeitsaufnahme-Charakteristik verschiedene Bereiche unterschiedlicher Dichte aufweisen. So ist beispielsweise aus der AU-B1-512002 ein absorbierender Artikel bekannt mit einem Saugkörper, der zwei unterschiedliche Dichten aufweist, wobei die Bereiche höherer oder niederer Dichte entweder linienförmig sich über den gesamten absorbierenden Artikel erstrecken oder kreisförmig ausgebildet sind. Weitere Ausgestaltungen von Saugkörpern mit Bereichen unterschiedlicher Dichte sind z.B. auch aus der EP-A 0291316, US-PS 4,435,178 und US-PS 3,965,904 bekannt.

Es ist nun Aufgabe der Erfindung, die bekannten Hygieneprodukte mit Saugkörpern mit Bereichen unterschiedlicher Dichte derart zu verbessern, daß die vorteilhafte Wirkung der Bereiche unterschiedlicher Dichten optimal ausnutzbar ist und gleichzeitig der Tragekomfort des Hygieneprodukts verbessert ist.

Die Aufgabe wird erfindungsgemäß gelöst durch die Merkmale des Patentanspruchs 1.

Die nach den kennzeichnenden Merkmalen des Anspruchs 1 vorgesehenen verdichteten Bereiche können in bekannter Weise die anfallende Flüssigkeit schneller aufsaugen und die übrigen, weniger verdichteten Bereiche die Flüssigkeit besser speichern. Darüber hinaus bewirkt die rechteckförmige Ausdehnung der verdichteten Bereiche, deren Längsseite sich quer zur Längsrichtung erstreckt und die in quer zur Längsrichtung des Saugkörpers verlaufenden Reihen angeordnet sind, eine erheblich verbesserte Anpassungmöglichkeit des Hygieneprodukts, beispielsweise einer Wegwerfwindel, an die Körperform des Trägers des Produktes. Gleichzeitig wird durch die verdichteten Bereiche die Stabilität des Hygieneprodukts erhöht, wobei der Saugkörper bei der Anpassung des Hygieneprodukts an die Körperform verbogen wird und dabei vorteilhafterweise weniger bricht als herkömmliche Produkte vergleichbarer Stabilität. Dies ergibt sich aus der rechtekkigen Form der verdichteten und in Reihe angeordneten Bereiche, deren Längsseite sich quer zur Längsrichtung des Saugkörpers erstreckt, da dadurch die verdichteten und weni-ger verdichteten Bereiche nach Art eines Scharnieres zusammenwirken.

Der Saugkörper weist eine Vielzahl verdichteter Bereiche auf, so daß sich die genannte Scharnierwirkung an vielen Stellen des Saugkörpers ergibt und dieser optimal an jede Körperform anpassbar ist, ohne daß die Gefahr besteht, daß der Saugkörper bricht. Dabei wird die Stabilität durch die vermehrte Anzahl verdichteter Bereiche noch erhöht.

Eine einfache Herstellung von Hygieneprodukten mit derartigen Saugkörpern ergibt sich, wenn die verdichteten Bereiche eine einheitliche Ausdehnung in Länge und Breite aufweisen.

Eine optimale Scharnierwirkung und damit Anpassung, insbesondere eine Anpassung an Krümmungen in Längsrichtung des Saugkörpers, die bei Hygieneprodukten am häufigsten auftritt, ergibt sich mit den Ausgestaltungen nach den Ansprüchen 3 bis 5.

Um die verdichteten Bereiche optimal über den Saugkörper zu verteilen und damit die an einer beliebigen Stelle anfallende Flüssigkeit schneller aufsaugbar ist, sind in der Ausgestaltung nach Anspruch 6 und 7 benachbarte Reihen verdichteter Bereiche auf Lücke versetzt angeordnet. Eine Ausgestaltung nach diesen Ansprüchen hat den weiteren Vorteil, daß die Flüssigkeit, die beim Verbiegen des Saugkörpers aus den weniger verdichteten und in einem höheren Maße die Flüssigkeit speichernden Bereichen ausgepresst wird, von den unmittelbar angrenzenden verdichteten Bereichen aufgenommen werden kann. Dies ist besonders vorteilhaft, wenn das Hygieneprodukt bereits eine große Flüssigkeitsmenge gespeichert hat und der Träger des Hygieneproduktes sich bewegt und dabei den an die Körperform angepaßten Saugkörper gezwungenermaßen verbiegt.

Eine vereinfachte Herstellung ergibt sich, wenn der Saugkörper gemäß Anspruch 8 nur zwei unterschiedliche Dichten aufweist.

Die Dichte der verdichteten Bereiche sollte gemäß Anspruch 9 etwa 10% mehr als die Dichte des übrigen Saugkörpers betragen, die bei etwa 0.1 bis 0.4 g/cm³ liegt.

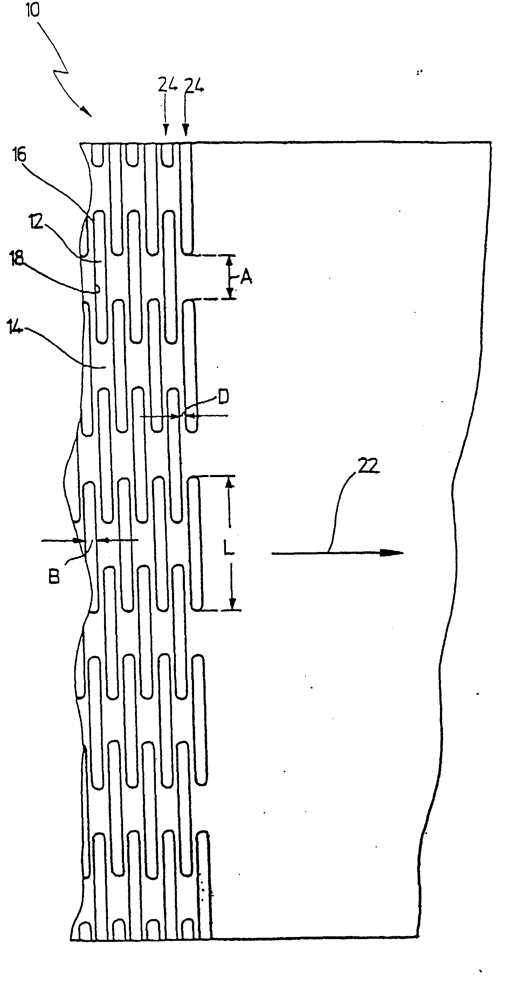

Im folgenden wird die Erfindung anhand des in der Zeichnung dargestellten Ausführungsbeispiels im einzelnen beschrieben. Die einzige Figur der Zeichnung zeigt eine Draufsicht auf einen Teil eines Saugkörpers eines Hygieneprodukts.

Der teilweise dargestellte Saugkörper 10 besteht im wesentlichen vorzugsweise aus Fasern, wie Zellulosefasern, die zur besseren Flüssigkeitsaufnahme behandelt sein können. Der Saugkörper 10 kann einschichtig oder mehrschichtig aufgebaut sein und kann superabsorbierendes Material in Form von Körnern oder Pulver in homogener oder inhomogener Verteilung aufweisen. In einem Saugkörper beispielsweise für eine Wegwerfwindel kann in der der Vorderseite des Trägers zugewandten Hälfte eine höhere Konzentration an superabsorbierendem Material vorgesehen sein, um den in dieser Hälfte größeren Flüssigkeitsanfall besser aufnehmen zu können.

Die Dicke des Saugkörpers 10 ist in der Regel gleichmäßig. Sie kann jedoch je nach Anwendungszweck in den Bereichen, in denen ein erhöhter Flüssigkeitsanfall auftritt, größer sein als in den übrigen Bereichen.

Der erfindungsgemäße Saugkörper 10 weist eine Vielzahl in der Zeichnung dargestellter, verdichteter Bereiche 12 auf, deren Dichte etwa 10% mehr beträgt als die Dichte der übrigen Bereiche 14, die bei etwa 0.1 bis 0.4 g/cm³ liegt.

Die verdichteten Bereiche 12 haben eine senkrecht zur Dikkenrichtung sich erstreckende, im wesentlichen rechteckige Ausdehnung 16 mit einer Breite B und einer Längsseite 18, deren Länge L größer ist als die Breite B und die quer zu einer durch Pfeil 22 dargestellten Längsrichtung des Saugkörpers 10 verläuft.

Die verdichteten Bereiche 12 haben eine einheitliche Ausdehnung in Länge L und Breite B mit einem Verhältnis Länge L zu Breite B von mindestens 2:1, vorzugsweise mindestens 5:1.

Einzelne verdichtete Bereiche 12 sind in Reihen 24 angeordnet, die quer zur Längsrichtung 22 des Saugkörpers 10 verlaufen. Der Abstand A zweier in einer Reihe 24 benachbarter, verdichteter Bereiche 12 ist größer als die Breite B der verdichteten Bereiche 12.

Benachbarte Reihen 24 verdichteter Bereiche 12 sind auf Lükke versetzt angeordnet. Die Distanz D zweier benachbarter Reihen 24 ist kleiner oder gleich der Breite B der verdichteten Bereiche 12.

Die Abmessungen für die Breite B, Länge L, Abstand A und Distanz D liegen vorzugsweise innerhalb der in der nachfolgenden Tabelle angegebenen Werte.

| | |
|---|---|
| Breite B | 1 mm bis 3 mm |
| Länge L | 10 mm bis 60 mm |
| Abstand A | 2 mm bis 20 mm |
| Distanz D | 0.2 mm bis 3 mm |

Die verdichteten Bereiche 12 können entweder nur auf einem Teil des Saugkörpers 10 oder auf dem gesamten Saugkörper 10 eingebracht sein. Der in der Zeichnung dargestellte Teil des Saugkörpers 10 weist zur besseren Darstellung nur auf einem Teilbereich die verdichteten Bereiche 12 auf.

Die verdichteten Bereiche können auf verschiedene, dem Fachmann bekannte Arten in den Saugkörper eingebracht werden. Beispielsweise kann zunächst ein Saugkörper mit gleichmäßiger Dicke und Dichte hergestellt werden, der dann in seiner Dicke lediglich in den zu verdichtenden Bereichen verpresst wird, so daß in diesen Bereichen die gewünschte Dichte erreicht wird. Diese Fertigungsmethode hat den Vorteil, daß der Saugkörper in den verdichteten Bereiche eine geringere Dicke hat als in den nicht verdichteten Bereichen. Dadurch entstehen in dem Hygieneprodukt zwischen dem Saugkörper und einer Saugkörperabdeckung Hohlräume, in denen bei einem sehr hohen, plötzlichen Flüssigkeitsanfall, die Flüssigkeit unmittelbar ohne Verzögerung aufgenommen wird und dann schnell von den verdichteten Bereichen aufgesaugt wird.

## Patentansprüche

1. Hygieneprodukt mit einem Fasern enthaltenden Saugkörper, mit stärker verdichteten Bereichen (12), die eine im wesentlichen rechteckförmige, sich senkrecht zur Dickenrichtung des Saugkörpers (10) und quer zu einer Längsrichtung (22) des Saugkörpers (10) erstreckende Ausdehnung haben, deren Länge (L) größer ist als deren Breite (B),
**dadurch gekennzeichnet, daß**
einzelne verdichtete Bereiche (12) in Reihen (24) angeordnet sind, die quer zur Längsrichtung (22) des Saugkörpers (10) verlaufen.

2. Hygieneprodukt nach Anspruch 1, dadurch gekennzeichnet, daß die stärker verdichteten Bereiche (12) in ihrer Abmessung einheitlich sind.

3. Hygieneprodukt nach Anspruch 1 oder 2, dadurch gekennzeichnet, daß das Verhältnis Länge (L) zu Breite (B) der verdichteten Bereiche (12) mindestens 2:1 beträgt.

4. Hygieneprodukt nach einem der vorhergehenden Ansprüche, dadurch gekennzeichnet, daß das Verhältnis Länge (L) zu Breite (B) mindestens 5:1 beträgt.

5. Hygieneprodukt nach einem der vorhergehenden Ansprüche, dadurch gekennzeichnet, daß der Abstand (A) zweier in einer Reihe (24) benachbarter, verdichteter Bereiche (12) größer ist als die Breite (B) der verdichteten Bereiche (12).

6. Hygieneprodukt nach einem der vorhergehenden Ansprüche, dadurch gekennzeichnet, daß benachbarte Reihen (24) verdichteter Bereiche (12) auf Lücke versetzt angeordnet sind.

7. Hygieneprodukt nach einem der vorhergehenden Ansprüche, dadurch gekennzeichnet, daß die Distanz (D) benachbarter Reihen (24) kleiner ist als die Breite (B) der verdichteten Bereiche (12).

8. Hygieneprodukt nach einem der vorhergehenden Ansprüche, dadurch gekennzeichnet, daß der Saugkörper (10) zwei unterschiedliche Dichten aufweist.

9. Hygieneprodukt nach Anspruch 8, dadurch gekennzeichnet, daß die Dichte des Saugkörpers (10) in nicht verdichteten Bereichen (14) 0.1 bis 0.4 g/cm³ beträgt und in den verdichteten Bereichen (12) um mindestens 10% größer ist.

10. Hygieneprodukt nach einem der vorhergehenden Ansprüche, dadurch gekennzeichnet, daß der Saugkörper (10) superabsorbierendes Material aufweist.

## Claims

1. A hygiene product having a fibrous absorbent body having regions (12) which are more tightly compressed and have a substantially rectangular elongation which extends perpendicularly with respect to the thickness of the absorbent body (10) and transversely with respect to a longitudinal direction (22) of the absorbent body (10), and whereof the length (L) is greater than the width (B), characterized in that individual compressed regions (12) are arranged in rows (24) running transversely with respect to the longitudinal direction (22) of the absorbent body (10).

2. A hygiene product according to Claim 1, characterized in that the more tightly compressed regions (12) have uniform dimensions.

3. A hygiene product according to Claim 1 or 2, characterized in that the ratio length (L) to width (B) of the compressed regions (12) is at least 2:1.

4. A hygiene product according to one of the preceding claims, characterized in that the ratio length (L) to width (B) is at least 5:1.

5. A hygiene product according to one of the preceding claims, characterized in that the spacing (A) between two compressed regions (12) adjacent in one row (24) is greater than the width (B) of the compressed regions (12).

6. A hygiene product according to one of the preceding claims, characterized in that adjacent rows (24) of compressed regions (12) are arranged spaced in offset manner.

7. A hygiene product according to one of the preceding claims, characterized in that the distance (D) between adjacent rows (24) is less than the width (B) of the compressed regions (12).

8. A hygiene product according to one of the preceding claims, characterized in that the absorbent body (10) has two different densities.

9. A hygiene product according to Claim 8, characterized in that the density of the absorbent body (10) is 0.1 to 0.4 g/cm³ in non-compressed regions (14) and is at least 10% greater in the compressed regions (12).

10. A hygiene product according to one of the preceding claims, characterized in that the absorbent body (10) contains super-absorbent material.

## Revendications

1. Produit d'hygiène comportant un corps absorbant contenant des fibres et comportant des zones fortement compressées (12), ayant une surface d'extension, qui est de forme sensiblement rectangulaire et s'étendant perpendiculairement à la direction de l'épaisseur du corps absorbant (10) et transversalement par rapport à une direction longitudinale (22) du corps absorbant (10) et dont la longueur (L) est plus grande que la largeur (B),
caractérisé en ce que :
des zones compressées (12) individuelles sont disposées selon des rangées (24) s'étendant transversalement par rapport à la direction longitudinale (22) du corps absorbant (10).

2. Produit d'hygiène selon la revendication 1, caractérisé en ce que les zones fortement compressées (12) sont unitaires en ce qui concerne leurs dimensions.

3. Produit d'hygiène selon la revendication 1 ou 2, caractérisé en ce que le rapport longueur (L) sur largeur (B) des zones compressées (12) est au moins égal à 2:1.

4. Produit d'hygiène selon l'une quelconque des revendications précédentes, caractérisé en ce que le rapport longueur (L) sur largeur (B) est au moins égal à 5:1.

5. Produit d'hygiène selon l'une quelconque des revendications précédentes, caractérisé en ce que la distance (A) entre deux zones compressées (12) voisines dans une rangée (24) est plus grande que la largeur (B) des zones compressées (12).

6. Produit d'hygiène selon l'une quelconque des revendications précédentes, caractérisé en ce que des rangées (24) voisines de zones compressées (12) sont disposées avec un décalage sur des vides.

7. Produit d'hygiène selon l'une quelconque des revendications précédentes, caractérisé en ce que la distance (D) entre deux rangées (24) voisines est plus petite que la largeur (B) des zones compressées (12).

8. Produit d'hygiène selon l'une quelconque des revendications précédentes, caractérisé en ce que le corps absorbant (10) présente deux densités différentes.

9. Produit d'hygiène selon la revendication 8, caractérisé en ce que la densité du corps absorbant (10) a une valeur comprise entre 0,1 et 0,4 g/cm³ dans les zones non compressées (14) et est supérieure d'au moins 10 % dans les zones compressées (12).

10. Produit d'hygiène selon l'une quelconque des revendications précédentes, caractérisé en ce que le corps absorbant (10) comprend une matière superaborbante.
